# EUROPÄISCHE PATENTSCHRIFT

(11) **EP 4 077 274 B1**
(45) Veröffentlichungstag und Bekanntmachung des Hinweises auf die Patenterteilung: **07.08.2024**
(21) Anmeldenummer: 20824936.7
(22) Anmeldetag: 15.12.2020
(51) Int. Cl.: C07C 263/10, C07C 265/14

(54) **VERFAHREN ZUR HERSTELLUNG VON DI- UND POLYISOCYANATEN DER DIPHENYLMETHANREIHE**
METHOD FOR THE PREPARATION OF DI- AND POLYISOCYANATES OF THE DIPHENYL METHANE SERIES
PROCÉDÉ DE FABRICATION DE DI- ET POLYISOCYANATES DE LA SÉRIE DU DIPHÉNYLMÉTHANE

(30) Priorität: 18.12.2019 EP 19217601; 29.10.2020 EP 20204702
(43) Veröffentlichungstag der Anmeldung: 26.10.2022
(73) Patentinhaber: Covestro Deutschland AG, 51373 Leverkusen (DE)
(72) Erfinder: WERSHOFEN, Stefan, 41065 Mönchengladbach (DE); LIU, Enke, 51375 Leverkusen (DE); HIELSCHER, Anke, 51063 Köln (DE); ADAMSON, Richard, 42799 Leichlingen (DE); SERRA, Ricardo, 40699 Erkrath (DE); MICHELE, Volker, 51065 Köln (DE); SPRIEWALD, Jürgen, 50769 Köln (DE)
(74) Vertreter: Levpat
(86) Internationale Anmeldenummer: PCT/EP2020/086261
(87) Internationale Veröffentlichungsnummer: WO 2021/122625

(56) Entgegenhaltungen:
- DE-A1- 2 847 243
- JP-A- 2004 027 160
- JP-A- H07 233 136

## Beschreibung

Die Erfindung betrifft ein Verfahren zur Herstellung von Di- und Polyisocyanaten der Diphenylmethanreihe, bei dem
**(i)** der in der Phosgenierung anfallende flüssige Produktstrom, **(ii)** das in einem gegebenenfalls vorhandenen Reaktor zur Carbaminsäurechlorid-Spaltung vorliegende Reaktionsgemisch, **(iii)** der einen solchen gegebenenfalls vorhandenen Reaktor zur Carbaminsäurechlorid-Spaltung verlassende flüssige Produkstrom, **(iv)** das in der Entphosgenierung vorliegende Reaktionsgemisch oder **(v)** der in der Entphosgenierung anfallende flüssige Produktstrom,
in einer Blasensäule oder in einer Bodenkolonne einstufig innerhalb einer Kontaktzeit von 1 min bis weniger als 30 min mit einem gasförmigen Chlorwasserstoffstrom behandelt wird. Der auf diese Weise behandelte Produktstrom bzw. das auf diese Weise behandelte Reaktionsgemisch wird direkt (d. h. insbesondere ohne weitere Behandlung mit einem Inertgas) dem nächstfolgenden Schritt der Reaktion bzw. Aufarbeitung zugeführt.

Aromatische Di- und Polyisocyanate sind wichtige und vielseitige Rohstoffe für die Polyurethanchemie. Hierbei sind neben Toluylendiisocyanat die Di- und Polyisocyanate der Diphenylmethanreihe (fortan summarisch MDI) technisch von besonderem Interesse. In allen großtechnisch relevanten Herstellverfahren wird MDI durch Phosgenierung der entsprechenden Di- und Polyamine der Diphenylmethanreihe (fortan summarisch MDA) gewonnen. MDA seinerseits wird durch säurekatalysierte Reaktion von Anilin und Formaldehyd gewonnen.

Ein im Zusammenhang mit der Herstellung oder Lagerung von MDI immer wieder auftretendes Problem ist dessen Dunkelfärbung. Es hat nicht an Versuchen gefehlt, diese zu unterbinden und ein möglichst "helles" MDI bereitzustellen. So beschreibt die japanische Patentanmeldung JP 2010/120870 A ein Verfahren zur Herstellung von MDI durch Phosgenierung von MDA in einem organischen Lösungsmittel, bei welchem der Reaktionsmischung nach erfolgter Phosgenierung und Abtrennung eines substanziellen Teils des eingesetzten Lösungsmittels (bis zu einer Rest-Konzentration im Bereich von 5 bis 35 Massen-%), und damit auch des Phosgens, Chlorwasserstoffgas zugesetzt wird, wobei die Temperatur während der Phosgenierung, der Lösungsmittelabtrennung und der Chlorwasserstoff-Behandlung in einem Bereich von 60 bis 130 °C gehalten wird. Die Behandlung mit Chlorwasserstoffgas nach Abtrennung eines substanziellen Teils des Lösungsmittels hat jedoch den Nachteil, dass sich das zu behandelnde Gemisch infolge vergleichsweise hoher Viskosität schwieriger handhaben lässt; daneben ist auch mit einer Verminderung der Effektivität der Behandlung sowie eine verstärkte Bildung von Ablagerungen zu erwarten.

Die japanische Patentanmeldung JP2010018534 (A) beschreibt ein Verfahren, bei dem zunächst ein aromatisches Polyamin durch säurekatalysierte Reaktion von Anilin und Formaldehyd oder Paraformaldehyd in einer ionischen Flüssigkeit gefolgt von Extraktion mit einem hydrophoben organischem Lösungsmittel erhalten wird. Nach Phosgenierung des so hergestellten aromatischen Polyamins und Abtrennung von Lösungsmittel und/oder überschüssigem Phosgen wird ein erstes Isocyanat erhalten. Dieses erste Isocyanat wird dann bei 60 bis 160 °C mit einem Chlorwasserstoffhaltigen zweiten Isocyanat, das durch Einmischen von Chlorwasserstoff in ein separat hergestelltes Isocyanat-Endprodukt (insbesondere kommerziell erhältliches MDI) erhalten wurde, vermischt. Die erhaltene Mischung wird im Anschluss auf Temperaturen im Bereich von 180 bis 230 °C erhitzt. Ein ähnliches Verfahren ist Gegenstand des japanischen Patents JP 5158199 B2. Diese Verfahrensweisen vermeiden die Eintragung von Chlorwasserstoffgas in den eigentlichen MDI-Herstellprozess bewusst. Sie müssen dafür erhebliche Nachteile wie etwa den aufwändigen Einsatz ionischer Flüssigkeiten in Kauf nehmen.

Die japanische Patentanmeldung JP H07233136 A befasst sich mit der Verbesserung der Farbe von MDI und auch insbesondere mit der Reduktion des Gehalts an sog. hydrolysierbarem Chlor in MDI. Zu diesem Zweck werden *zwischen* der Entphosgenierkolonne (zur Entfernung überschüssigen Phosgens) und der Lösungsmittelabtrennkolonne (zur Entfernung des eingesetzten Lösungsmittels) mehrere Apparate zur Behandlung des rohen MDI mit Chlorwasserstoff bei erhöhter Temperatur geschaltet. Bei dieser nach der Entphosgenierung durchgeführten mehrstufigen Chlorwasserstoffbehandlung steigt die Temperatur von Stufe zu Stufe, insbesondere von 60 bis 140 °C in einer ersten Stufe (beispielsweise 30 min bei 115 °C) auf 150 bis 170 °C in einer zweiten Stufe (beispielsweise 3 min bei 160 °C). Die beschriebene mehrstufige Chlorwasserstoffbehandlung ist aufwändig und erfordert Investitionen in mehrere zusätzliche Apparate. Eine *einstufige* Chlorwasserstoffbehandlung nach der Entphosgenierung wird als nachteilig dargestellt (siehe Vergleichsbeispiel 1). Eine einstufige Chlorwasserstoffbehandlung *ohne* eine *separate* Entphosgenierung (d. h. hier soll die Chlorwasserstoffbehandlung zusätzlich zur Farbaufhellung auch zur Austreibung von Phosgen dienen) wird ebenfalls als nachteilig dargestellt (siehe Vergleichsbeispiel 2).

Die japanische Patentanmeldung JP 2004/027160 A beschreibt ein Verfahren zur Herstellung von MDI, bei welchem eine MDI-Rohlösung zunächst mit Chlorwasserstoffgas und dann mit einem Inertgas wie Stickstoff durchströmt wird. In allen konkret offenbarten Ausführungsformen, insbesondere auch in allen Beispielen (sowie in allen Vergleichsbeispielen), ist die mit Chlorwasserstoffgas behandelte MDI-Rohlösung eine Lösung, die durch Entphosgenierung des Reaktionsprodukts der Phosgenierung von MDA mit Phosgen in Gegenwart eines Lösungsmittels erhalten wurde. Die Inertgasbehandlung kann entweder unmittelbar nach der Behandlung mit Chlorwasserstoffgas oder nach einer zwischengeschalteten Lösungsmittelabtrennung erfolgen. Hierdurch wird der Gehalt an hydrolysierbarem Chlor im MDI reduziert. Die Schrift kommt ausdrücklich zu der Schlussfolgerung, dass eine alleinige Chlorwasserstoffbehandlung ohne nachfolgende Inertgasbehandlung nachteilig ist. Daher ist davon auszugehen, dass in der Schrift offenbarte Verfahrensparameter der Chlorwasserstoffbehandlung auf das beanspruchte kombinierte Verfahren umfassend eine Behandlung mit Chlorwasserstoff *und* Inertgas ausgelegt sind. Die zusätzliche Behandlung mit einem Inertgas ist jedoch aufwändig, verursacht Kosten und führt zu einer erhöhten Abgasfracht.

Die japanische Patentanmeldung JP 2006/104166 A beschreibt eine Methode zur Verringerung des Gehalts an hydrolysierbarem Chlor, bei welcher rohes MDI (das nur einer Abtrennung von Lösungsmittel und überschüssigem Phosgen unterworfen wurde) bei einer Temperatur in einem Bereich von 150 bis 250 °C bei einem Druck von 5 bis 25 kPa in Gegenwart eines Metalloxids mit einem Gas durchströmt wird. Als geeignete Gase werden Stickstoff, Chlorwasserstoff, Bromwasserstoff, Kohlenstoffmonoxid und Kohlenstoffdioxid beschrieben. Der Einsatz von Metalloxiden ist aufwändig und verursacht Kosten. Ferner müssen die Metalloxide wieder abgetrennt werden.

Die deutsche Patentanmeldung DE 28 47 243 A1 beschreibt ein Verfahren zur Herstellung eines organischen Isocyanats, bei dem ein organisches Amin mit Phosgen umgesetzt wird, um ein Reaktionsprodukt zu liefern, welches das entsprechende organische Isocyanat enthält, bei welchem Chlorwasserstoffgas durch dieses Reaktionsprodukt in Anwesenheit eines inerten organischen Lösungsmittels geleitet wird, wobei das in diesem Reaktionsprodukt enthaltene Gas entfernt wird und die in dem Reaktionsprodukt enthaltenen sauren und hydrolysierbaren chlorhaltigen Substanzen minimalisiert werden. Bei dem Reaktionsprodukt handelt es sich um das rohe flüssige Verfahrensprodukt der Umsetzung des Amins mit Phosgen (welche in Gegenwart eines Lösungsmittels erfolgt). Die bei der Umsetzung ebenfalls gebildete Gasphase, die im Wesentlichen aus Chlorwasserstoffgas und nicht umgesetztem Phosgen besteht, kann nach weitgehender Absorption überschüssigen Phosgens in einem Lösungsmittel direkt für den Zweck des Hindurchleitens von Chlorwasserstoffgas durch das Reaktionsprodukt verwendet werden. Dabei wird im Reaktionsprodukt noch gelöstes Phosgen abgetrieben. Ein separater, von der Chlorwasserstoffbehandlung verschiedener Schritt des Entphosgenierens wird nicht beschrieben. (Es wird lediglich die Austreibung gelösten Phosgens mittels Hindurchleiten von Stickstoff als Stand der Technik, von dem sich das in DE 28 47 243 A1 beschriebene Verfahren abgrenzen will, beschrieben.) Die Chlorwasserstoffgasbehandlung erfolgt bevorzugt in einer Füllkörperkolonne bei einer Temperatur von 140 °C bis 230 °C über eine Zeitdauer von 0,5 bis 5 Stunden. Andere Reaktionsapparate oder kürzere Kontaktzeiten werden in der Schrift nicht offenbart.

Wie geschildert weisen die Verfahren des Standes der Technik, die sich eine Farbverbesserung (bzw. eine Verringerung des Gehalts an hydrolysierbarem Chlor) des MDI zum Ziel gesetzt haben, verschiedene Nachteile auf. Es bestand daher noch Bedarf an Verbesserungen auf diesem Gebiet. Insbesondere wäre es wünschenswert, eine effiziente Methode zur Farbaufhellung von MDI zur Verfügung zu haben, die mit möglichst geringem apparativen Aufwand in bestehende MDI-Produktionsanlagen integriert werden kann, die möglichst wenig zusätzliche Abgasfracht verursacht und auf den Einsatz systemfremder Katalysatoren (wie Metalloxide) verzichten kann. Es wäre weiterhin insbesondere wünschenswert, eine effiziente Methode zur Farbaufhellung und/oder Verringerung des Chlorgehaltes (ausgedrückt als Gesamtchlorgehalt und/oder als hydrolysierbares Chlor) von MDI bereitzustellen, die mit möglichst wenig zusätzlichen Reagenzien auskommt.

Diesem Bedarf Rechnung tragend ist ein Gegenstand der vorliegenden Erfindung ein **Verfahren zur Herstellung von Di- und Polyisocyanaten der Diphenylmethanreihe,** umfassend die Schritte:

### - Reaktion -

(I) Umsetzen von Di- und Polyaminen der Diphenylmethanreihe mit Phosgen in Gegenwart eines Lösungsmittels in einem Phosgenierungsreaktor unter Erhalt **(a)** eines Chlorwasserstoff und Phosgen (sowie gegebenenfalls verdampftes Lösungsmittel) enthaltenden **ersten gasförmigen Produktstroms** und **(b)** eines Di- und Polyisocyanate der Diphenylmethanreihe, Phosgen und Lösungsmittel (sowie gegebenenfalls noch Carbaminsäurechloride) enthaltenden **ersten flüssigen Produktstroms;** und
(II) optional, Umsetzen des ersten flüssigen Produktstroms in einem Reaktor zur Carbaminsäurechlorid-Spaltung, wobei sich in dem Reaktor zur Carbaminsäurechlorid-Spaltung ein erstes Reaktionsgemisch bildet, das in **(a)** einen Chlorwasserstoff und Phosgen (sowie gegebenenfalls verdampftes Lösungsmittel) enthaltenden **zweiten gasförmigen Produktstrom** und **(b)** einen Isocyanat, Phosgen und Lösungsmittel enthaltenden **zweiten flüssigen Produktstrom** getrennt wird; sowie

### - Aufarbeitung -

(III) Abtrennen von Phosgen aus dem ersten flüssigen Produktstrom oder, bei Durchführung von Schritt (II), aus dem zweiten flüssigen Produktstrom, in einer Entphosgenierungsvorrichtung, wobei sich in der Entphosgenierungsvorrichtung ein zweites Reaktionsgemisch bildet, das in **(a)** einen Chlorwasserstoff und Phosgen (sowie gegebenenfalls verdampftes Lösungsmittel) enthaltenden **dritten gasförmigen Produktstrom** und **(b)** einen Isocyanat und Lösungsmittel enthaltenden **dritten flüssigen Produktstrom** (der gegenüber dem ersten bzw. zweiten flüssigen Produktstrom an Phosgen abgereichert ist) getrennt wird;
(IV) Abtrennen von Lösungsmittel aus dem dritten flüssigen Produktstrom unter Erhalt **(a)** eines Lösungsmittel enthaltenden **vierten gasförmigen Produktstroms** und **(b)** eines Isocyanat enthaltenden **vierten flüssigen Produktstroms,** wobei dem Abtrennen des Lösungsmittels keine Inertgasbehandlung des erhaltenen vierten flüssigen Produktstroms folgt;
(V) optional, Abtrennen eines Teils der Diisocyanate der Diphenylmethanreihe aus dem vierten flüssigen Produktstrom unter Erhalt **(a)** eines Diisocyanate der Diphenylmethanreihe enthaltenden **fünften gasförmigen Produkstroms,** der kondensiert wird, und (b) eines gegenüber dem vierten flüssigen Produktstrom an Polyisocyanaten der Diphenylmethanreihe angereicherten **fünften flüssigen Produktstroms;**
(VI) optional, Abtrennen von Phosgen aus dem ersten und dritten gasförmigen Produktstrom, bei Durchführung von Schritt (II) aus dem ersten, zweiten und dritten gasförmigen Produktstrom, unter Erhalt **(a)** eines Chlorwasserstoff enthaltenden **sechsten gasförmigen Produktstroms** und **(b)** eines Phosgen (sowie Lösungsmittel, sofern solches in den gasförmigen Produktströmen enthalten war) enthaltenden sechsten **flüssigen Produktstroms;**
   wobei
(VII)
   (a) der in Schritt (I) anfallende erste flüssige Produktstrom mit einem gasförmigen Chlorwasserstoffstrom behandelt und dann direkt dem Schritt (II) oder dem Schritt (III) zugeführt wird, oder
   (b) das im Reaktor zur Carbaminsäurechlorid-Spaltung aus Schritt (II) gebildete erste Reaktionsgemisch mit einem gasförmigen Chlorwasserstoffstrom behandelt wird, oder
   (c) der in Schritt (II) anfallende zweite flüssige Produktstrom mit einem gasförmigen Chlorwasserstoffstrom behandelt und dann direkt dem Schritt (III) zugeführt wird, oder
   (d) das in der Entphosgenierungsvorrichtung aus Schritt (III) gebildete zweite Reaktionsgemisch mit einem gasförmigen Chlorwasserstoffstrom behandelt wird, oder
   (e) der in Schritt (III) anfallende dritte flüssige Produktstrom mit einem gasförmigen Chlorwasserstoffstrom behandelt und dann direkt dem Schritt (IV) zugeführt wird,
wobei das Behandeln mit dem gasförmigen Chlorwasserstoffstrom einstufig in einer Blasensäule oder in einer Bodenkolonne durchgeführt wird, wobei eine Kontaktzeit des gasförmigen Chlorwasserstoffstroms mit dem zu behandelnden (ersten, zweiten oder dritten) flüssigen Produktstrom oder dem zu behandelnden (ersten oder zweiten) Reaktionsgemisch im Bereich von 1 min bis weniger als 30 min eingestellt wird.

Unter einem *gasförmigen Chlorwasserstoffstrom* wird erfindungsgemäß ein Gasstrom verstanden, der Chlorwasserstoff in einem auf seine Gesamtmasse bezogenen Anteil von mindestens 90 Massen-%, bevorzugt mindestens 95 Massen-%, besonders bevorzugt mindestens 98 Massen-% und ganz besonders bevorzugt mindestens 99 Massen-%, umfasst. Dabei kann es sich um einen reinen gasförmigen Chlorwasserstoffstrom handeln, d. h. um einen Strom, der - gegebenenfalls abgesehen von unbedeutenden Spurenanteilen produktionsbedingter Verunreinigungen, die jedoch regelmäßig nicht mehr als 2 000 ppm ausmachen und daher nicht ins Gewicht fallen - nur Chlorwasserstoffgas umfasst. Es kann sich aber auch um einen gasförmigen Chlorwasserstoffstrom handeln, der Fremdstoffe, insbesondere Phosgen, gasförmige Lösungsmittel und/oder inerte Gase, in signifikanten Anteilen umfasst, zum Beispiel ein Chlorwasserstoffstrom, der im erfindungsgemäßen Verfahren selbst anfällt. Ein derartiger Anteil an Fremdstoffen beträgt jedoch maximal 10 Massen-%, bevorzugt maximal 5 Massen-%, besonders bevorzugt maximal 2 Massen-% und ganz besonders bevorzugt maximal 1 Massen-%, bezogen auf die Gesamtmasse des gasförmigen Chlorwasserstoffstroms.

Die erfindungsgemäß in Schritt (VII) vorzunehmende Behandlung *"mit einem gasförmigen Chlorwasserstoffstrom"* (fortan auch kurz *erfindungsgemäße Chlorwasserstoffbehandlung)* kann grundsätzlich mit einem beliebigen unter (VII) (a) bis (VII) (e) genannten Produktstrom bzw. Reaktionsgemisch durchgeführt werden. Im Rahmen der vorliegenden Erfindung bezeichnet der Ausdruck *"mit einem gasförmigen Chlorwasserstoffstrom behandelt* ... *wird"* das Durchströmen von Chlorwasserstoffgas durch den zu behandelnden Produktstrom bzw. durch das zu behandelnde Reaktionsgemisch.

Die erfindungsgemäße Chlorwasserstoffbehandlung erfolgt *"einstufig* ", d. h. der zu behandelnde Produktstrom bzw. das zu behandelnde Reaktionsgemisch einer gegebenen Temperatur wird in einem Schritt kontinuierlich mit Chlorwasserstoffgas einer gegebenen Temperatur durchströmt (und nicht wie in manchen Verfahren des Standes der Technik in mehreren Schritten in hintereinandergeschalteten Apparaten mit sukzessive steigender Temperatur).

Die erfindungsgemäße Chlorwasserstoffbehandlung kommt im Gegensatz zu manchen Verfahren des Standes der Technik ohne die Zugabe von Katalysatoren (wie Metalloxiden) aus; daher wird Schritt (VII) insbesondere ohne Zugabe eines Katalysators durchgeführt.

Nach Durchführung der erfindungsgemäßen Chlorwasserstoffbehandlung wird der auf diese Weise behandelte Produktstrom bzw. das auf diese Weise behandelte Reaktionsgemisch direkt dem nächstfolgenden Schritt der Reaktion oder Aufarbeitung zugeführt, wobei mit *"direkt* ... *zugeführt"* gemeint ist, dass keine weiteren zwischengeschalteten Verfahrensschritte durchgeführt werden. Der nächstfolgende Schritt ist dabei im Fall der Chlorwasserstoffbehandlung des ersten, zweiten oder dritten flüssigen Produktstroms der Schritt (II), (III) bzw. (IV). Im Fall der Chlorwasserstoffbehandlung des ersten oder zweiten Reaktionsgemisches ist der nächstfolgende Schritt ein Teilschritt des Schrittes (II) bzw. (III), nämlich die Trennung des ersten bzw. zweiten Reaktionsgemisches in eine gasförmige und eine flüssige Phase. Erfolgt also die erfindungsgemäße Chlorwasserstoffbehandlung beispielsweise gemäß der Variante (VII)(a) am ersten flüssigen Produktstrom, so wird dieser nach der erfindungsgemäßen Chlorwasserstoffbehandlung direkt (oder unmittelbar; d. h. insbesondere ohne weitere Behandlung mit einem Inertgas, wie sie in manchen Verfahren des Standes der Technik beschrieben wird) dem Schritt (II) oder - bei Wegfall dieses optionalen Schrittes - dem Schritt (III) zugeführt. In diesem Zusammenhang unterscheidet die in dieser Erfindung verwendete Terminologie nicht in besonderer Weise zwischen einem *unbehandelten* (ohne bzw. vor erfindungsgemäßer Chlorwasserstoffbehandlung) und einem *behandelten* (nach erfindungsgemäßer Chlorwasserstoffbehandlung) Produktstrom (bzw. Reaktionsgemisch); beide können beispielsweise als *"erster flüssiger Produktstrom"* bezeichnet werden.

Es versteht sich von selbst, dass die Varianten (VII)(b) und (VII)(c) nur bei Durchführung von Schritt (II) anwendbar sind. Eine Durchführung von Schritt (II) impliziert aber nicht zwingend die Anwendung einer dieser Varianten. Es ist ohne weiteres möglich, Schritt (II) durchzuführen und die Chlorwasserstoffbehandlung beispielsweise gemäß (VII)(a) durchzuführen.

Unter einer *"Blasensäule"* (auch *Blasensäulenreaktor* genannt) wird im Rahmen der vorliegenden Erfindung im Einklang mit dem in der Fachwelt üblichen Sprachgebrauch ein verfahrenstechnischer Apparat für Gas/Flüssigkeit-Prozesse verstanden, wobei in der Blasensäule in eine Flüssigkeit (hier: *der zu behandelnde Produktstrom bzw. das zu behandelnde Reaktionsgemisch)* ein Gas (hier: *der gasförmige Chlorwasserstoffstrom)* eingebracht wird, das in Blasen durch die Flüssigkeit perlt und dabei eine Phasengrenzfläche zwischen Gas und Flüssigkeit erzeugt. Die Begasung erfolgt vorzugsweise durch ein gelochtes Bodenblech, durch das das Gas strömt. Die Flüssigkeit kann im Gleichstrom oder im Gegenstrom geführt werden.

Unter einer *"Bodenkolonne"* wird im Rahmen der vorliegenden Erfindung im Einklang mit dem in der Fachwelt üblichen Sprachgebrauch eine Destillationskolonne verstanden, die horizontale Einbauten, so genannte Böden, aufweist. Die Böden sind Platten mit Durchtrittsöffnungen. Als derartige Böden eignen sich so genannte *Sieb-, Glocken- oder Ventilböden,* auf denen Flüssigkeit (hier: *der zu behandelnde Produktstrom bzw. das zu behandelnde Reaktionsgemisch)* steht. Durch spezielle Schlitze oder Löcher wird eine Gasphase (hier: *der gasförmige Chlorwasserstoffstrom)* in die Flüssigkeit eingeperlt, so dass eine Sprudelschicht entsteht. Auf jedem dieser Böden stellt sich ein neues temperaturabhängiges Gleichgewicht zwischen der Flüssig- und Gasphase ein. Im Idealfall entspricht ein Boden einer theoretischen Trennstufe.

In den beigefügten Zeichnungen zeigen
- **FIG. 1**: Eine Darstellung der Schritte (I) bis (VI) des erfindungsgemäßen Verfahrens;
- **FIG. 2**: Eine Darstellung einer bevorzugten Umsetzung des Schrittes (VII) des erfindungsgemäßen Verfahrens.

Es folgt zunächst eine Kurzzusammenfassung verschiedener möglicher Ausführungsformen.

In einer **ersten Ausführungsform** der Erfindung wird Schritt (VII) gemäß der Variante (VII)(a), (VII)(c) oder (VII)(e) durchgeführt.

In einer **zweiten Ausführungsform** der Erfindung wird Schritt (VII) gemäß der Variante (VII)(a) oder (VII)(c) durchgeführt.

In einer **dritten Ausführungsform** der Erfindung, die mit allen anderen Ausführungsformen kombiniert werden kann, enthält der dritte flüssige Produktstrom Phosgen in einem auf seine Gesamtmasse bezogenen Massenanteil im Bereich von 0,001 ppm bis 1000 ppm, bevorzugt im Bereich von im Bereich von 0,01 ppm bis 100 ppm.

In einer **vierten Ausführungsform** der Erfindung, die mit allen anderen Ausführungsformen kombiniert werden kann, enthält der in Schritt (VII) eingesetzte gasförmige Chlorwasserstoffstrom Phosgen in einem auf seine Gesamtmasse bezogenen Massenanteil im Bereich von 1 ppm bis 10000 ppm, bevorzugt 10 ppm bis 10000 ppm, besonders bevorzugt im Bereich von im Bereich von 10 ppm bis 1000 ppm.

In einer **fünften Ausführungsform** der Erfindung, die mit allen anderen Ausführungsformen kombiniert werden kann, insbesondere mit der vierten Ausführungsform, umfasst das Verfahren den Schritt (VI), wobei der sechste gasförmige Produktstrom teilweise oder vollständig in Schritt (VII) als gasförmiger Chlorwasserstoffstrom verwendet wird (dem weiterer Chlorwasserstoff zugemischt werden kann, aber nicht muss).

In einer **sechsten Ausführungsform** der Erfindung, die mit allen anderen Ausführungsformen kombiniert werden kann, wird das molare Verhältnis von Chlorwasserstoff, der in dem in Schritt (VII) eingesetzten gasförmigen Chlorwasserstoffstrom enthalten ist, zu Isocyanatgruppen, die in dem in Schritt (VII) behandelten Reaktionsgemisch oder Produktstrom enthalten sind, n(HCl)/n(NCO), auf einen Wert im Bereich von 0,1 bis 2,0, bevorzugt 0,1 bis 1,0, besonders bevorzugt 0,1 bis 0,5, eingestellt.

In einer **siebten Ausführungsform** der Erfindung, die mit allen anderen Ausführungsformen kombiniert werden kann, sofern diese nicht auf eine Gleichstromfahrweise in Schritt (VII) beschränkt sind (d. h. mit Ausnahme der nachfolgend beschriebenen achten Ausführungsform), werden in Schritt (VII)
- der gasförmige Chlorwasserstoffstrom und
- der zu behandelnde Produktstrom bzw. das zu behandelnde Reaktionsgemisch im Gegenstrom geführt.

In einer **achten Ausführungsform** der Erfindung, die mit allen anderen Ausführungsformen kombiniert werden kann, sofern diese nicht auf eine Gegenstromfahrweise in Schritt (VII) beschränkt sind (d. h. mit Ausnahme der siebten Ausführungsform), werden in Schritt (VII)
- der gasförmige Chlorwasserstoffstrom und
- der zu behandelnde Produktstrom bzw. das zu behandelnde Reaktionsgemisch
im Gleichstrom geführt.

In einer **neunten Ausführungsform** der Erfindung, die mit allen anderen Ausführungsformen kombiniert werden kann, wird die Behandlung mit dem gasförmigen Chlorwasserstoffstrom in Schritt (VII) bei einer Temperatur des zu behandelnden Produktstroms bzw. des zu behandelnden Produktgemisches im Bereich von 70 °C bis 135 °C, bevorzugt 80 °C bis 135 °C, besonders bevorzugt 90 °C bis 135 °C, und bei einer Temperatur des eingesetzten Chlorwasserstoffgasstroms im Bereich von 20 °C bis 135 °C, bevorzugt 25 °C bis 135 °C, besonders bevorzugt 30 °C bis 135 °C, durchgeführt.

In einer **zehnten Ausführungsform** der Erfindung, die mit allen anderen Ausführungsformen kombiniert werden kann, insbesondere mit der zwölften Ausführungsform, wird die Behandlung mit dem gasförmigen Chlorwasserstoffstrom in Schritt (VII) isotherm durchgeführt.

In einer **elften Ausführungsform** der Erfindung, die mit allen anderen Ausführungsformen kombiniert werden kann, wird in Schritt (VII) eine Kontaktzeit des gasförmigen Chlorwasserstoffstroms mit dem zu behandelnden (ersten, zweiten oder dritten) flüssigen Produktstrom bzw. dem zu behandelnden (ersten oder zweiten) Reaktionsgemisch im Bereich von 1 min bis 25 min, bevorzugt 1 min bis 20 min, besonders bevorzugt 2 min bis 20 min, ganz besonders bevorzugt 4 min bis 20 min, eingestellt.

In einer **zwölften Ausführungsform** der Erfindung, die mit allen anderen Ausführungsformen kombiniert werden kann, erfolgt die Behandlung mit dem gasförmigen Chlorwasserstoffstrom in Schritt (VII) bei einem Druck im Bereich von Umgebungsdruck bis 5,00 bar_{(abs.)}, bevorzugt bei einem Druck im Bereich von 1,50 bar_{(abs.)} bis 3,50 bar_{(abs.)}.

Die zuvor kurz geschilderten Ausführungsformen und weitere mögliche Ausgestaltungen der Erfindung werden im Folgenden näher erläutert. Dabei können die geschilderten Ausführungsformen und weiteren möglichen Ausgestaltungen beliebig unter- und miteinander kombiniert werden, sofern sich aus dem Zusammenhang nichts anderes ergibt.

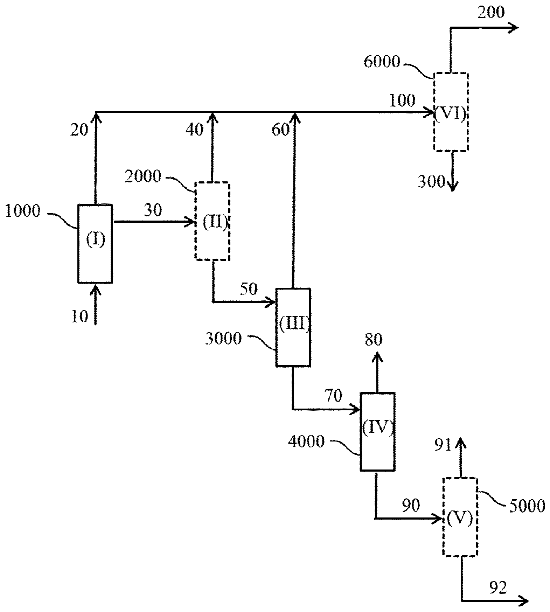

Die eigentliche Herstellung des MDI gemäß den Schritten (I) bis (VI) kann, sofern die erfindungsgemäße Chlorwasserstoffbehandlung gemäß Schritt (VII) gewährleistet ist, erfolgen wie aus dem Stand der Technik an sich bekannt. **FIG. 1** zeigt schematisch und stark vereinfacht eine dazu geeignete Produktionsanlage. Die Bezugszeichen haben dabei die folgende Bedeutung.

### Apparate (in der breitesten Ausführungsform der Erfindung optionale Apparate sind gestrichelt gezeichnet):

| | |
|---|---|
| 1000 | Phosgenierungsreaktor; |
| 2000 | Reaktor zur Carbaminsäurechlorid-Spaltung (optional); |
| 3000 | Entphosgenierungsvorrichtung; |
| 4000 | Vorrichtung zur Lösungsmittelabtrennung; |
| 5000 | Vorrichtung zur partiellen Abtrennung von Diisocyanaten der Diphenylmethanreihe (optional und bevorzugt); |
| 6000 | Vorrichtung zur Abtrennung von Phosgen aus im Verfahren anfallenden Phosgen-haltigen Gasströmen (optional und bevorzugt). |

### Stoffströme:

| | |
|---|---|
| 10 | Gemisch aus MDA, Phosgen und Lösungsmittel; |
| 20 | erster gasförmiger Produktstrom; |
| 30 | erster flüssiger Produktstrom; |
| 40 | zweiter gasförmiger Produktstrom; |
| 50 | zweiter flüssiger Produktstrom; |
| 60 | dritter gasförmiger Produktstrom; |
| 70 | dritter flüssiger Produktstrom; |
| 80 | vierter gasförmiger Produktstrom; |
| 90 | vierter flüssiger Produktstrom; |
| 91 | fünfter gasförmiger Produktstrom; |
| 92 | fünfter flüssiger Produktstrom; |
| 100 | Gemisch des ersten, zweiten (sofern vorhanden) und dritten Gasstroms; |
| 200 | sechster gasförmiger Strom; |
| 300 | sechster flüssiger Strom. |

Die Herstellung von MDI lässt sich exemplarisch wie folgt zusammenfassen:
a) Kernvorgang des Schritts (I): MDA wird im Phosgenierungsreaktor (1000) mit Phosgen in einem Lösungsmittel zu MDI umgesetzt. Zu diesem Zweck werden bevorzugt zunächst Lösungen von MDA in dem Lösungsmittel und von Phosgen in dem Lösungsmittel hergestellt und im Phosgenierungsreaktor oder einer stromaufwärts zu diesem angeordneten Mischvorrichtung vermischt. Das im Phosgenierungsreaktor erhaltene rohe Verfahrensprodukt wird in einen flüssigen, Roh-MDI und Lösungsmittel (sowie Phosgen und Chlorwasserstoff) enthaltenden Strom (30) und einen gasförmigen, Phosgen und Chlorwasserstoff (sowie Lösungsmittel) enthaltenden Strom (20) aufgetrennt. Als Phosgenierungsreaktoren eignen sich verschiedene Reaktortypen, z. B. Turmreaktoren (auch als *Phosgeniertürme* bezeichnet), Rohrreaktoren oder Rührkessel. Es ist möglich und verlässt den Rahmen der vorliegenden Erfindung nicht, mehrere Phosgenierungsreaktoren in Reihe und/oder parallel zu schalten, z. B. eine Reihenschaltung in Form einer Rührkesselkaskade.
b) Kernvorgang des Schrittes (II): Sofern erforderlich wird der im Phosgenierungsreaktor erhaltene Strom 30 in einem Reaktor zur Carbaminsäurechlorid-Spaltung (2000) (dem sog. *CSC-Spalter)* weiter unter Chlorwasserstoffabspaltung (abgeführt über Strom 40) umgesetzt. Dieser Schritt kann dann durchgeführt werden, wenn der im Phosgenierungsreaktor erhaltene Strom 30 noch substanzielle Anteile an Carbaminsäurechlorid enthält. Schritt (II) liefert einen flüssigen Strom 50, der weitgehend bis vollständig von Carbaminsäurechlorid befreit ist.
c) Kernvorgang des Schrittes (III): Abtrennung weiteren bei der Reaktion entstandenen Chlorwasserstoffes zusammen mit nicht umgesetztem Phosgen (abgeführt über Strom 60) aus dem Strom 30 bzw. dem Strom 50 in der Entphosgenierungsvorrichtung (3000) (dem sog. *Entphosgenierer*). Dabei wird ein flüssiger MDI-Strom 70 erhalten, der weitgehend bis vollständig von Chlorwasserstoff und Phosgen befreit ist.
d) Kernvorgang des Schrittes (IV): Abtrennung von Lösungsmittel aus dem in der Entphosgenierungsvorrichtung (3000) erhaltenen flüssigen Strom 70 in der Vorrichtung zur Lösungsmittelabtrennung (4000) (der sog. *Lösungsmittelkolonne).* Dabei wird ein von Lösungsmittel weitgehend bis vollständig befreiter Roh-MDI-Strom 90 erhalten. Das abgetrennte Lösungsmittel fällt zunächst gasförmig als Strom 80 an.
e) Kernvorgang des Schrittes (V): Sofern gewünscht, partielle Abtrennung von Diisocyanaten der Diphenylmethanreihe (fortan auch MMDI genannt) aus dem in der Vorrichtung zur Lösungsmittelabtrennung (4000) erhaltenen Roh-MDI-Strom 90 in der Vorrichtung zur partiellen Abtrennung von Diisocyanaten der Diphenylmethanreihe (5000) (der sog. *Polymerabtrennung*). Dabei werden ein zunächst gasförmig anfallender MMDI-Strom (91) und ein flüssiger MDI-Strom (der im Vergleich zu Strom 90 an MMDI abgereichert ist) erhalten.
f) Kernvorgang des Schrittes (VI): Bevorzugt, Abtrennen von Phosgen aus den im Verfahren anfallenden Phosgen-haltigen Gasströmen (in FIG. 1 die Ströme 20, 40 und 60) in der Vorrichtung zur Abtrennung von Phosgen (6000). Sollte der in der Lösungsmittelabtrennung anfallende Strom 80 noch zu große Mengen an Phosgen und Chlorwasserstoff enthalten, so ist es bevorzugt, diesen Strom 80 nach Kondensation einer Strippung zu unterziehen und den dabei erhaltenen Strippgasstrom ebenfalls der Vorrichtung zur Abtrennung von Phosgen (6000) zuzuführen. In Schritt (VI) fallen ein Lösungsmittel-haltiger Phosgenstrom (300) und ein Chlorwasserstoff-haltiger Gasstrom (200) an. Diese HCl-Phosgen-Trennung kann durch Absorption in einem Lösungsmittel oder (nach vorangehender Verflüssigung) durch Destillation erfolgen.

Die kontinuierliche Produktion des MDI **in a)** erfolgt in einer Reaktionsstrecke nach einem aus dem Stand der Technik bekannten Verfahren. Geeignete Verfahren sind beispielsweise in EP 2 077 150 B1, EP 1 616 857 A1, EP 1 873 142 A1, EP 0 716 079 B1 oder EP 0 314 985 B1 beschrieben. Konzentrationen und Mengenströme der Edukte MDA und Phosgen werden dabei jedoch bevorzugt so gewählt, dass sich in der Mischzone ein molares Verhältnis von Phosgen zu primären Aminogruppen von 1,1 : 1 bis 30 : 1, besonders bevorzugt von 1,25 : 1 bis 3 : 1, einstellt. Alle Verfahren für die Produktion von MDI liefern ein rohes Verfahrensprodukt, das in eine Flüssigphase (30), enthaltend neben dem gewünschten Isocyanat gelösten Chlorwasserstoff, überschüssiges gelöstes Phosgen und Lösungsmittel, und eine Gasphase (20), enthaltend Chlorwasserstoffgas, überschüssiges gasförmiges Phosgen und gasförmiges Lösungsmittel, zerfällt bzw. aufgetrennt wird. Erfindungsgemäß umfasst ist auch eine Ausführungsform, bei der der erhaltene Roh-Isocyanat-Strom 30 vor der weiteren Verarbeitung in c) eine Vorrichtung zur Spaltung von Carbaminsäurechlorid in **b)** durchläuft.

Die weitere Abtrennung von Chlorwasserstoff und Phosgen aus dem flüssigen Roh-Isocyanat-Strom 30 bzw. 50 in der sog. Entphosgenierungsvorrichtung in c) kann nach einem beliebigen aus dem Stand der Technik bekannten Verfahren erfolgen, zum Beispiel wie in EP 1 854 783 A2, WO 2004/031132 A1 oder WO 2004/056756 A1 beschrieben. Bevorzugt erfolgt die Entphosgenierung durch Destillation in einer Kolonne, der sog. Entphosgenierkolonne. Am Kopf der Entphosgenierkolonne fällt der dritte gasförmige Produktstrom an, während der dritte flüssige Produktstrom am Sumpf erhalten wird. Die Entphosgenierung gemäß Schritt (III) wird vorzugsweise so ausgestaltet, dass der die Entphosgenierungsvorrichtung verlassende dritte flüssige Produktstrom Phosgen in einem auf seine Gesamtmasse bezogenen Massenanteil im Bereich von 0,001 ppm bis 1000 ppm, bevorzugt im Bereich von im Bereich von 0,01 ppm bis 100 ppm, enthält. Die zuvor geschilderten Verfahrensweisen ermöglichen dies dem Fachmann ohne Weiteres.

Die weitere Abtrennung von Lösungsmittel aus so erhaltenen flüssigen Isocyanat-Strom 70 in der Vorrichtung zur Lösungsmittelabtrennung **in d)** kann nach einem beliebigen aus dem Stand der Technik bekannten Verfahren erfolgen, bevorzugt wie in EP 1 854 783 B1 beschrieben.

Die optionale Abtrennung von Diisocyanaten der Diphenylmethanreihe aus dem im Schritt (IV) erhaltenen flüssigen Isocyanat-Strom 90 **in e)** kann nach einem beliebigen aus dem Stand der Technik bekannten Verfahren erfolgen. Geeignete Verfahren sind z. B. beschrieben in EP 1 854 783 A2 und EP 1 506 957 A1, oder auch in EP 1 475 367 A1 oder WO 2012/065927 A1.

Die HCl-Phosgen-Trennung **in f)** kann nach einem beliebigen aus dem Stand der Technik bekannten Verfahren erfolgen, bevorzugt wie in DE-A-10260084, EP 1 849 767 A1 oder EP 2 093 215 A1 beschrieben.

Die **erfindungsgemäße Chlorwasserstoffbehandlung gemäß Schritt (VII)** umfasst eine der möglichen Varianten (a) bis (e). Bevorzugt ist die Variante (VII)(a). Die Durchführung der erfindungsgemäßen Chlorwasserstoffbehandlung an dieser Stelle des Verfahrens hat eine Reihe von Vorteilen. So ist bei Behandlung des ersten flüssigen Produktstroms die thermische Vorbelastung des zu behandelnden Materials im Vergleich am geringsten, wodurch die Tendenz zu Nebenreaktionen bei der Chlorwasserstoffbehandlung, die zu unerwünschten (teilweise auch wieder farbgebenden) Nebenprodukten führen können, gering ausfällt. Farbverbesserung und Reduktion des Chlorgehaltes im Endprodukt sind in dieser Ausführungsform besonders ausgeprägt. Ferner wird bei Behandlung des ersten flüssigen Produktstroms mit Chlorwasserstoff zumindest ein Teil des in diesem Strom gelösten Phosgens ausgestrippt, wodurch nachfolgende Prozessschritte von der Aufgabe der Phosgenabtrennung zumindest teilweise entlastet werden.

Werden in Schritt (I) bei Durchführung des Schritts (VII) in der bevorzugten Variante (a) n parallel geschaltete Phosgenierungsreaktoren eingesetzt, wobei *n* eine natürliche Zahl im Bereich von 2 bis 10 ist, sodass mehrere flüssige Produktströme (30-1, 30-2, ... 30-*n*) der Art des ersten flüssigen Produktstroms (30) anfallen, so können diese flüssigen Produktströme (30-1, 30-2, ... 30-*n*) vor Durchführung des Schrittes (VII) vereinigt werden. Es ist aber auch möglich, diese Produktströme getrennt dem Schritt (VII) zu unterziehen, nämlich insbesondere dann, wenn sich die einzelnen flüssigen Produktströme (30-1, 30-2, ... 30-*n*) hinsichtlich ihrer Isomeren- und/oder Homologenverteilung unterscheiden, was im Alltag einer MDI-Produktionsanlage nicht ungewöhnlich ist. Selbstverständlich ist es auch möglich, die *n* flüssigen Produktströme in Gruppen zusammenzufassen, die jeweils unterschiedlich behandelt werden können. Zum Beispiel können bei sechs parallel geschalteten Phosgenierungsreaktoren, also n = 6, die flüssigen Produktströme 30-1 und 30-2 einerseits und die flüssigen Produktströme 30-3 und 30-4 andererseits jeweils gemeinsam dem Schritt (VII) zugeführt werden, während die flüssigen Produktströme 30-5 und 30-6 jeweils einzeln dem Schritt (VII) zugeführt werden. Die Beschränkung auf eine einzige *Variante* schließt also nicht notwendigerweise aus, dass mehrere *Apparate* zur Durchführung des Schritts (VII) vorgesehen sind, denn es wird trotzdem jeder der Ströme (30-1, 30-2, ... 30-*n*) im Sinne der Erfindung *einstufig* mit Chlorwasserstoff behandelt.

Betrifft die erfindungsgemäße Chlorwasserstoffbehandlung ein in einem Apparat vorliegendes Reaktionsgemisch (Varianten (VII)(b) und (VII)(d)), so wird der Chlorwasserstoffstrom direkt in diesen Apparat eingeleitet (beispielsweise in den unteren Teil der Entphosgenierungsvorrichtung). In diesem Fall ist dann der entsprechende Apparat (*Reaktor zur Carbaminsäurechlorid-Spaltung* bzw. *Entphosgenierungsvorrichtung*) erfindungsgemäß als *Blasensäule* oder *Bodenkolonne* auszugestalten. Ferner sind dann die Reaktionsbedingungen (insbesondere Temperatur und Druck) so zu wählen, dass die in dieser Ausführungsform notwendigerweise gleichzeitig ablaufenden Prozesse (Carbaminsäurechlorid-Spaltung und Chlorwasserstoffbehandlung in Variante (VII)(b) bzw. Entphosgenierung und Chlorwasserstoffbehandlung in Variante (VII)(d)) innerhalb der erfindungsgemäß vorgesehenen Kontaktzeit für die Chlorwasserstoffbehandlung ablaufen können. Für den Fachmann ist es ein Leichtes, entsprechende Auslegungskriterien aufzufinden.

Betrifft die erfindungsgemäße Chlorwasserstoffbehandlung einen flüssigen Produktstrom, der einen Apparat verlässt (Varianten (VII)(a), (VII)(c) und (VII)(e)), so erfolgt die erfindungsgemäße Chlorwasserstoffbehandlung in einer eigens dafür vorgesehenen Vorrichtung, die zwischen dem Apparat, aus dem der zu behandelnde Produktstrom austritt, und dem Apparat des nächstfolgenden Schritts angeordnet ist. Als derartige Vorrichtungen zur Behandlung (= Durchströmen) einer Flüssigkeit (hier: der entsprechende flüssige Produktstrom) mit einem Gasstrom (hier: Chlorwasserstoffgasstrom) werden erfindungsgemäß *Blasensäulen* oder Destillationskolonnen, die als *Bodenkolonnen* ausgeführt sind, eingesetzt. Blasensäulen können entweder ohne Einbauten verwendet werden oder, bevorzugt, mit Einbauten (z. B. statische Systeme wie Lochbleche oder dynamische Mischersysteme), um die Rückvermischung zu reduzieren und/oder eine Redispergierung von flüssiger Phase und Gasphase zu ermöglichen und/oder zu unterstützen; gegebenenfalls können auch statische und dynamische Systeme kombiniert werden.

Dabei kann die erfindungsgemäße Chlorwasserstoffbehandlung z. B. in Blasensäulen im Gegenstrom oder im Gleichstrom durchgeführt werden. *Gegenstrom* bedeutet in der Terminologie der vorliegenden Erfindung, dass bei senkrecht angeordneter Blasensäule der flüssige Produktstrom den Apparat von oben nach unten durchströmt, während der Gasstrom den Apparat von unten nach oben durchströmt. Gleichstrom bedeutet in der Terminologie der vorliegenden Erfindung, dass bei senkrecht angeordneter Blasensäule sowohl der flüssige Produktstrom als auch der Gasstrom den Apparat von unten durchströmen. Bei der Durchführung der erfindungsgemäßen Chlorwasserstoffbehandlung in Blasensäulen ist die Gegenstromfahrweise bevorzugt.

In jedem Fall ist es bevorzugt, die Behandlung mit dem gasförmigen Chlorwasserstoffstrom in Schritt (VII) bei einer Temperatur des zu behandelnden Produktstroms bzw. des zu behandelnden Produktgemisches im Bereich von 70 °C bis 135 °C, bevorzugt 80 °C bis 135 °C, besonders bevorzugt 90 °C bis 135 °C, und bei einer Temperatur des eingesetzten Chlorwasserstoffstroms im Bereich von 20 °C bis 135 °C, bevorzugt 25 °C bis 135 °C, besonders bevorzugt 30 °C bis 135 °C, durchzuführen. Es ist weiterhin bevorzugt, insbesondere in Verbindung mit Einhaltung der zuvor genannten Temperaturen, die erfindungsgemäße Chlorwasserstoffbehandlung isotherm, also unter möglichster Konstanthaltung der Temperatur durch Thermostatisierung (d. h., abhängig von der insgesamt infolge der ablaufenden physikalischen und chemischen Vorgänge vorherrschenden Wärmetönung, unter Wärmezufuhr durch Beheizen oder Wärmeabfuhr durch Kühlen, hier insbesondere unter Wärmezufuhr, optional unterstützt durch Isolierung der verwendeten Apparatur gegen Wärmeverluste an die Umgebung), durchzuführen.

Die Behandlung mit dem gasförmigen Chlorwasserstoffstrom in Schritt (VII) erfolgt bei Umgebungsdruck oder bei im Vergleich zu Umgebungsdruck erhöhtem Druck, bevorzugt bei einem Druck im Bereich von Umgebungsdruck, insbesondere 1,01 bar_{(abs.)}, bis 5,00 bar _{(abs.)}, besonders bevorzugt bei einem Druck von 1,50 bar_{(abs.)} bis 3,50 bar_{(abs.)}.

Unabhängig von der genauen Art der Verfahrensführung ist es vorgesehen, eine Kontaktzeit des gasförmigen Chlorwasserstoffstroms mit dem zu behandelnden Produktstrom im Bereich von 1 min bis weniger als 30 min, bevorzugt 1 min bis 25 min, besonders bevorzugt 1 min bis 20 min, ganz besonders bevorzugt 2 min bis 20 min, außerordentlich ganz besonders bevorzugt 4 min bis 20 min, einzustellen.

Die erfindungsgemäße Chlorwasserstoffbehandlung kann grundsätzlich mit Chlorwasserstoff aus den unterschiedlichsten Quellen durchgeführt werden. Neben reinem Chlorwasserstoff ist auch der Einsatz von als Koppelprodukt chemischer Reaktionen, insbesondere der MDI-Herstellung selbst, anfallender Chlorwasserstoff denkbar. Insbesondere im letztgenannten Fall kann es sein, dass der Chlorwasserstoff noch geringe Mengen an Phosgen enthält, insbesondere in einem auf seine Gesamtmasse bezogenen Massenanteil im Bereich von 1 ppm bis 10000 ppm, bevorzugt von 10 ppm bis 10000 ppm, besonders bevorzugt im Bereich von im Bereich von 10 ppm bis 1000 ppm. Vollkommen überraschend wurde gefunden, dass sich dieser Phosgenanteil nicht nachteilig auswirkt, weder auf die eigentliche Chlorwasserstoffbehandlung noch auf die Qualität des hergestellten MDI.

In der bevorzugten Ausgestaltung der Erfindung unter Durchführung von Schritt (VI) kann vorteilhafterweise der in diesem Schritt anfallende sechste gasförmige Produktstrom teilweise oder vollständig in Schritt (VII) als gasförmiger Chlorwasserstoffstrom verwendet werden (wobei weiterer Chlorwasserstoff zugemischt werden kann, aber nicht muss). Dies hat den großen Vorteil, dass der Einsatz von Chlorwasserstoff aus externen Quellen (Quellen außerhalb des MDI-Verfahrens) minimiert oder sogar völlig verzichtbar wird.

Unabhängig von der genauen Quelle des Chlorwasserstoffs und der Art des zu behandelnden Produktstroms bzw. Reaktionsgemisches ist es bevorzugt, in Schritt (VII) ein molares Verhältnis von Chlorwasserstoff, der in dem in Schritt (VII) einzusetzenden gasförmigen Chlorwasserstoffstrom enthalten ist, zu Isocyanatgruppen, die in dem in Schritt (VII) zu behandelnden Reaktionsgemisch bzw. Produktstrom enthalten sind, n(HCl)/n(NCO), einzustellen, das in einem Bereich von 0,1 bis 2,0, bevorzugt 0,1 bis 1,0, besonders bevorzugt 0,1 bis 0,5, liegt.

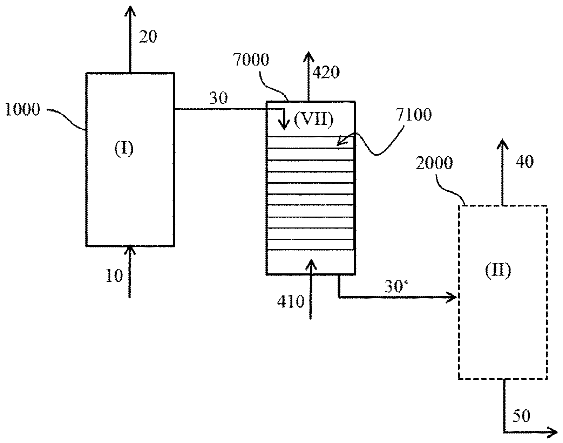

Die besonders bevorzugte Ausgestaltung der Erfindung unter Durchführung der erfindungsgemäßen Chlorwasserstoffbehandlung in der Variante (VII)(a) ist in **FIG. 2** gezeigt. Die Bezugszeichen, die auch FIG. 1 verwendet werden, haben die gleiche Bedeutung wie dort. Die weiteren Bezugszeichen bedeuten Folgendes:

### Apparate:

| | |
|---|---|
| 7000: | Vorrichtung (= Bodenkolonne) zur Behandlung (= Durchströmen) des ersten flüssigen Produktstroms (30) mit einem Chlorwasserstoffgasstrom im Gegenstrom; |
| 7100: | Böden; |

### Stoffströme:

| | |
|---|---|
| 30`: | Mit Chlorwasserstoffgas behandelter erster flüssiger Produktstrom (30); |
| 410: | Chlorwasserstoffgasstrom; |
| 420: | Nach Durchlaufen der Kolonne 7000 verbleibender Chlorwasserstoffgasstrom. Dieser wird bevorzugt (in FIG. 2 nicht gezeigt) mit den Strömen 20, 40 (sofern vorhanden) und 60 vereint, sodass Strom 420 Bestandteil des Stroms 100 wird. |

Insgesamt zeichnet sich die Erfindung daher durch die folgenden Vorteile aus:
- Deutliche Reduktion der Farbwerte.
- Signifikante Reduktion des Chlorgehaltes (ausgedrückt als Gesamtchlorgehalt und/oder als hydrolysierbares Chlor).
- Entgegen dem Stand der Technik ist es nicht erforderlich, vor der Behandlung mit Chlorwasserstoff das Phosgen aus der flüssigen Phase abzutrennen.
- Es ist nicht erforderlich, reinen Chlorwasserstoff zu verwenden; vielmehr sind auch Chlorwasserstoff-haltige Prozessströme mit einem Restgehalt an Phosgen geeignet. Dadurch kann auf eine aufwändige Reinigung des Chlorwasserstoff-Gasstroms bzw. auf die Verwendung von extern zugeführtem, reinem Chlorwasserstoff verzichtet werden.
- Bei der bevorzugten Variante (VII) (a) wird zudem durch die geringe thermische Vorbelastung des Materials die Tendenz zu Nebenreaktionen minimiert, die ansonsten zu unerwünschten (teilweise auch farbgebenden) Nebenprodukten führen könnten. Es wird eine ausgeprägte Farbverbesserung und Reduktion des Chlorgehaltes im Endprodukt beobachtet.
- Bei der bevorzugten Variante (VII) (a) wird zumindest ein Teil des Phosgens aus der flüssigen Phase durch Chlorwasserstoff ausgestrippt; dadurch werden nachfolgende Prozessschritte von der Aufgabe der Phosgenabtrennung zumindest teilweise entlastet.

### Beispiele:

In den nachfolgenden Beispielen sind zwei verschiedene Apparaturen für die Chlorwasserstoffbehandlung verwendet worden, die nachstehend beschrieben werden.

### A) Bodenkolonne (Reaktivrektifikation):

Die aus Glas gefertigte Kolonne mit einem Innendurchmesser von 50 mm und einer Länge von 700 mm war mit 10 Ventilböden versehen. Der gesamte Flüssigkeitsholdup auf den Böden der Kolonne betrug ca. 200 ml. Die Bodenkolonne wurde bei Atmosphärendruck betrieben und war mit einem elektrischen Heizband umwickelt, um Wärmeabstrahlung an die Umgebung zu vermeiden; die Temperatur dieser Begleitheizung war auf 120 °C eingestellt. Der flüssige Eingangsstrom wurde oberhalb des obersten Bodens auf die Kolonne aufgegeben, während der gasförmige Eingangsstrom unterhalb des untersten Bodens der Kolonne zugeführt wurde (die Bodenkolonne wurde also in der Terminologie der vorliegenden Erfindung *im Gegenstrom* betrieben).

Beide Eingangsströme wurden mittels Wärmeaustauschern temperiert, bevor sie in die Kolonne eintraten. Die Temperatur des Gasstroms betrug ca. 60 °C bis 80 °C. Die Temperatur des flüssigen Eingangsstroms betrug ca. 125 °C.

Der gasförmige Ausgangsstrom wurde am Kopf der Kolonne entnommen und durch einen Wärmeaustauscher geleitet, um kondensierbare Bestandteile (im Wesentlichen Monochlorbenzol, MCB) aufzufangen. Der flüssige Ausgangsstrom wurde am Boden der Kolonne unterhalb des Zugabepunktes des gasförmigen Eingangsstroms entnommen und in einem auf 60 °C temperierten Doppelmantelgefäß aufgefangen, dem die Proben zur weiteren Aufarbeitung und Analytik entnommen wurden.

### B) Blasensäule

Die aus Glas gefertigte Doppelmantel-Blasensäule mit einem Innendurchmesser von 50 mm und einer Länge von 700 mm war mit insgesamt 10 Lochböden mit jeweils 2,5 % freier Fläche (Lochfläche) und einem Ringbegaser versehen. Der untere Teil der Blasensäule bis zu einer Höhe von 150 mm war durch einen Lochboden abgetrennt und diente als Temperierzone. 50 mm darüber war der Ringbegaser angeordnet, und darüber jeweils im Abstand von 50 mm zueinander die weiteren 9 Lochböden. Das Volumen der Blasensäule oberhalb des Ringbegasers betrug ca. 1000 ml. Der zur Temperierung der Blasensäule genutzte Doppelmantel (Wärmeträger: Silikonöl) war in zwei Sektionen geteilt, deren Temperatur unabhängig voneinander eingestellt werden konnte. Die untere Sektion umfasste den Bereich unterhalb des Lochbodens, der unter dem Ringbegaser angeordnet war, die obere Sektion umfasste den größeren Teil der Blasensäule, der darüber lag.

Der gasförmige Eingangsstrom wurde mit einer Temperatur von ca. 20 °C bis 25 °C (Umgebungstemperatur) über den Ringbegaser der Blasensäule zugeführt. Der gasförmige Ausgangsstrom wurde am Kopf der Blasensäule entnommen und durch einen Wärmeaustauscher geleitet, um kondensierbare Bestandteile (im Wesentlichen MCB) aufzufangen.

Die Blasensäule wurde im Gleichstrom betrieben. Der flüssige Eingangsstrom wurde zunächst mittels eines Wärmeaustauschers auf 100 °C temperiert und dann am Boden der Kolonne unterhalb des Zugabepunktes des gasförmigen Eingangsstroms am Boden der Temperierzone in die Blasensäule gegeben. Der flüssige Ausgangsstrom wurde am Kopf der Blasensäule ca. 70 mm oberhalb des obersten Lochbodens entnommen und in einem auf 60 °C temperierten Doppelmantelgefäß aufgefangen, dem die Proben zur weiteren Aufarbeitung und Analytik entnommen wurden. (Ein Betrieb der Blasensäule im Gegenstrom ist ebenfalls ohne weiteres möglich. Zu diesem Zweck kann der flüssige Eingangsstrom (nach Temperierung mittels eines Wärmeaustauschers auf 100 °C wie im Gleichstrombetrieb) ca. 70 mm oberhalb des obersten Lochbodens auf die Blasensäule aufgegeben werden. Der flüssige Ausgangsstrom kann am Boden der Kolonne unterhalb des Zugabepunktes des gasförmigen Eingangsstroms am Boden der Temperierzone entnommen und wie für Gleichstrombetrieb beschrieben weiter behandelt werden.)

Die Blasensäule wurde bei erhöhtem Druck, der mittels Manometer überwacht wurde, betrieben. Die Druckhaltung erfolgte manuell über Nadelventile im Ausgang des flüssigen und des gasförmigen Ausgangsstroms. (Ein Betrieb der Blasensäule bei Umgebungsdruck ist jedoch ohne weiteres ebenfalls möglich.)

Unabhängig von der verwendeten Apparatur dienten als gasförmige Einsatzstoffe entweder Chlorwasserstoff (aus einer Druckgasflasche), Stickstoff oder Phosgen, oder Gemische der genannten Gase. Als flüssige Einsatzstoffe dienten im Zuge der Phosgenierung von MDA in MCB erhaltene Proben, d. h. - in der Terminologie der vorliegenden Erfindung - ein erster flüssiger Produktstrom (d. h. der Austrag eines Phosgenierreaktors, u. a. enthaltend MCB, MDI, korrespondierende Carbaminsäurechloride und Phosgen) bzw. ein dritter flüssiger Produktstrom (d. h. der Austrag einer Entphosgenierstufe, u. a. enthaltend MCB und MDI; der Strom war praktisch frei von Carbaminsäurechlorid und Phosgen). (Nähere Einzelheiten können den konkreten Beschreibungen der einzelnen Beispiele in Tabelle 1 entnommen werden.)

Die nach der Behandlung erhaltenen Proben - sowie unbehandelte Referenzproben - wurden nach einem standardisierten Prozedere aufgearbeitet. Zunächst wurde im Wasserstrahlvakuum (ca. 20 mbar_{(abs.)}) die Hauptmenge des in der Probe enthaltenen Lösungsmittels MCB abdestilliert, wobei die Sumpftemperatur 100 °C nicht überschritt. Danach wurde der flüssige Sumpf (im Wesentlichen MDI) in eine kleinere Destillationsapparatur überführt, in der unter reduziertem Druck (ca. 2 bis 3 mbar_{(abs.)}) die Lösungsmittelreste abdestilliert wurden, und zwar bis zu einer Sumpftemperatur von 190 °C. Danach wurde die Destillation abgebrochen und der Destillationssumpf (MDI) innerhalb weniger Minuten auf Raumtemperatur abgekühlt.

Das erhaltene Produkt (MDI) wurde mittels folgender Parameter analytisch charakterisiert: NCO-Wert, Viskosität, Farbwerte (Extinktion bei 430 nm und 520 nm), bei einem Teil der Versuche zusätzlich der Gesamtchlor-Gehalt.

Zur Bestimmung des NCO-Wertes wurde eine Probe des Isocyanates mit überschüssigem Di-*n-*butylamin umgesetzt, gefolgt von einer Rücktitration des überschüssigen Amins mit einer Salzsäure-Maßlösung.

Die dynamische Viskosität wurde unter Verwendung eines Kugelfall-Viskosimeters bei 25 °C gemessen.

Zur Bestimmung der Farbwerte (E430, E520) wurde eine Lösung von 1,00 g Isocyanat in 50 ml MCB hergestellt. Ein Teil dieser Lösung wurde in eine Küvette mit 10 mm Schichtdicke überführt, und die Extinktion bei 430 nm bzw. 520 nm wurde mit Hilfe eines Farbmessgeräts LICO 690 der Firma Hach Lange gegen MCB als Referenz gemessen.

Der Gesamtchlor-Gehalt (Total-Chlor, TC) wurde mittels Röntgenfluoreszenzanalyse bestimmt.

**Tabelle 1: Gegenüberstellung der Versuchsbedingungen und Resultate der Beispiele**

| **Beispiel** | **Art der Behandlung** | **Flüssiger Eingangsstrom** | | **Gasförmiger Eingangsstrom** | | **Stöchiometrie** | **Verweilzeit der Flüssigkeit (ca-Werte)** | **Betriebsbedingungen der Kolonne bzw. Blasensäule** | | **Produkteigenschaften** | | | | |
|---|---|---|---|---|---|---|---|---|---|---|---|---|---|---|
| | | **Quelle** | **Flussrate** | **Quelle** | **Flussrate** | | | **Temperatur** | **Druck** | **NCO** | **Viskosität bei 25°C** | **E430** | **E520** | **TC** |
| | | | **[kg/h]** | | **[Nl/h]** | **mol HCl / mol NCO** | **[min]** | **[°C]** | **[bar abs.]** | **[Gew.-%]** | **[mPas]** | | | **[ppm]** |
| 1 (erfindungsgemäß) | Bodenkolonne | Austrag Entphosgenierer (ca. 21 % MDI) | 4 | HCl | 320 | 2 | 4 | 125 | UD | 31,6 | 110 | 0,048 | 0,008 | 1441 |
| 2 (erfindungsgemäß) | Bodenkolonne | Austrag Entphosgenierer (ca. 21 % MDI) | 4 | HCl enthaltend 1 % Phosgen | 320 | 2 | 4 | 125 | UD | 31,6 | 107 | 0,053 | 0,009 | 1439 |
| 3 (Vergleich) | unbehandelt | Austrag Phosgenierreaktor (ca. 21 % MDI) | | | | | | | | 32,0 | 88 | 0,107 | 0,016 | 2319 |
| 4 (erfindungsgemäß) | Bodenkolonne | Austrag Phosgenierreaktor (ca. 21 % MDI) | 2 | HCl | 80 | 1 | 8 | 125 | UD | 31,8 | 86 | 0,037 | 0,006 | 1564 |
| 5 (Vergleich) | unbehandelt | Austrag Entphosgenierer (ca. 21 % MDI) | | | | | | | | 32,0 | 83 | 0,098 | 0,013 | 2462 |
| 6 (erfindungsgemäß) | Bodenkolonne | Austrag Entphosgenierer (ca. 21 % MDI) | 2 | HCl | 80 | 1 | 8 | 125 | UD | 31,8 | 85 | 0,054 | 0,008 | 1932 |
| 7 (Vergleich) | Bodenkolonne | Austrag Phosgenierreaktor (ca. 21 % MDI) | 2 | N2 | 80 | 0 | 8 | 125 | UD | 31,7 | 111 | 0,139 | 0,022 | n.b. |
| 8 (Vergleich) | Bodenkolonne | Austrag Phosgenierreaktor (ca. 21 % MDI) | 2 | HCl:N2 25:75 | 80 | 0,25 | 8 | 125 | UD | 31,7 | 110 | 0,108 | 0,015 | n.b. |
| 9 (Vergleich) | Bodenkolonne | Austrag Phosgenierreaktor (ca. 21 % MDI) | 2 | HCl:N2 50:50 | 80 | 0,5 | 8 | 125 | UD | 31,7 | 113 | 0,082 | 0,011 | n.b. |
| 10 (Vergleich) | Bodenkolonne | Austrag Phosgenierreaktor (ca. 21 % MDI) | 2 | HCl:N2 75:25 | 80 | 0,75 | 8 | 125 | UD | 31,6 | 115 | 0,054 | 0,010 | n.b. |
| 11 (erfindungsgemäß) | Bodenkolonne | Austrag Phosgenierreaktor (ca. 21 % MDI) | 2 | HCl | 80 | 1 | 8 | 125 | UD | 31,5 | 115 | 0,037 | 0,008 | n.b. |
| 12 (Vergleich) | unbehandelt | Austrag Phosgenierreaktor (ca. 21 % MDI) | | | | | | | | 31,9 | 119 | 0,118 | 0,016 | n.b. |
| 13 (erfindungsgemäß) | Bodenkolonne | Austrag Phosgenierreaktor (ca. 21 % MDI) | 2 | HCl | 40 | 0,5 | 8 | 125 | UD | 31,7 | 120 | 0,065 | 0,010 | n.b. |
| 14 (erfindungsgemäß) | Bodenkolonne | Austrag Phosgenierreaktor (ca. 21 % MDI) | 2 | HCl | 80 | 1 | 8 | 125 | UD | 31,7 | 116 | 0,043 | 0,007 | n.b. |
| 15 (Vergleich) | unbehandelt | Austrag Phosgenierreaktor (ca. 21 % MDI) | | | | | | | | 31,9 | 132 | 0,122 | 0,016 | 2323 |
| 16 (erfindungsgemäß) | Blasensäule | Austrag Phosgenierreaktor (ca. 21 % MDI) | 4 | HCl | 80 | 0,5 | 15 | 100 | 3 | 31,8 | 132 | 0,059 | 0,011 | 1789 |

| | | | | | | | | | | | | | | |
|---|---|---|---|---|---|---|---|---|---|---|---|---|---|---|
| UD = Umgebungsdruck (ca. 1 bar_{(abs.)}); n. b. = nicht bestimmt | | | | | | | | | | | | | | |

Die Ergebnisse der Beispiele 1 bis 16 machen Folgendes deutlich:
Die erfindungsgemäße Behandlung mit gasförmigem Chlorwasserstoff führt zu einer deutlichen Verbesserung der Farbe des erhaltenen MDI (Erniedrigung der Extiktionswerte bei 430 nm und 520 nm). Zudem wird noch eine Reduzierung des Gesamtchlor-Gehaltes erreicht. Dabei ist es wesentlich, dass Chlorwasserstoffgas eingesetzt wird, da Vergleichsversuche gezeigt haben, dass die Verwendung eines Inertgases wie Stickstoff keinen positiven Effekt zeigt. Im Gegensatz zu den Aussagen im Teilen des Standes der Technik erlaubt das erfindungsgemäße Verfahren auch die Gegenwart eines gewissen Anteils von Phosgen im eingesetzten gasförmigem Chlorwasserstoff und/oder in dem zu behandelnden Produktstrom bzw. Reaktionsgemisch: Die Beispiele zeigen, dass es keineswegs erforderlich ist, überschüssiges Phosgen aus der Phosgenierung abzutrennen, bevor die Behandlung mit gasförmigem Chlorwasserstoff durchgeführt wird. Ganz im Gegenteil wird das Ergebnis bei Durchführung der Chlorwasserstoffbehandlung am flüssigen Rohprodukt der Phosgenierung sogar noch verbessert.

## Patentansprüche

1. Verfahren zur Herstellung von Di- und Polyisocyanaten der Diphenylmethanreihe, umfassend die Schritte:
(I) Umsetzen von Di- und Polyaminen der Diphenylmethanreihe mit Phosgen in Gegenwart eines Lösungsmittels in einem Phosgenierungsreaktor unter Erhalt (a) eines Chlorwasserstoff und Phosgen enthaltenden ersten gasförmigen Produktstroms und (b) eines Di- und Polyisocyanate der Diphenylmethanreihe, Phosgen und Lösungsmittel enthaltenden ersten flüssigen Produktstroms;
(II) optional, Umsetzen des ersten flüssigen Produktstroms in einem Reaktor zur Carbaminsäurechlorid-Spaltung, wobei sich in dem Reaktor zur Carbaminsäurechlorid-Spaltung ein erstes Reaktionsgemisch bildet, das in (a) einen Chlorwasserstoff und Phosgen enthaltenden zweiten gasförmigen Produktstrom und (b) einen Isocyanat, Phosgen und Lösungsmittel enthaltenden zweiten flüssigen Produktstrom getrennt wird;
(III) Abtrennen von Phosgen aus dem ersten flüssigen Produktstrom oder, bei Durchführung von Schritt (II), aus dem zweiten flüssigen Produktstrom, in einer Entphosgenierungsvorrichtung, wobei sich in der Entphosgenierungsvorrichtung ein zweites Reaktionsgemisch bildet, das in (a) einen Chlorwasserstoff und Phosgen enthaltenden dritten gasförmigen Produktstrom und (b) einen Isocyanat und Lösungsmittel enthaltenden dritten flüssigen Produktstrom getrennt wird;
(IV) Abtrennen von Lösungsmittel aus dem dritten flüssigen Produktstrom unter Erhalt (a) eines Lösungsmittel enthaltenden vierten gasförmigen Produktstroms und (b) eines Isocyanat enthaltenden vierten flüssigen Produktstroms, wobei dem Abtrennen des Lösungsmittels keine Inertgasbehandlung des erhaltenen vierten flüssigen Produktstroms folgt;
(V) optional, Abtrennen eines Teils der Diisocyanate der Diphenylmethanreihe aus dem vierten flüssigen Produktstrom unter Erhalt (a) eines Diisocyanate der Diphenylmethanreihe enthaltenden fünften gasförmigen Produkstroms, der kondensiert wird, und (b) eines gegenüber dem vierten flüssigen Produktstrom an Polyisocyanaten der Diphenylmethanreihe angereicherten fünften flüssigen Produktstroms;
(VI) optional, Abtrennen von Phosgen aus dem ersten und dritten gasförmigen Produktstrom, bei Durchführung von Schritt (II) aus dem ersten, zweiten und dritten gasförmigen Produktstrom, unter Erhalt (a) eines Chlorwasserstoff enthaltenden sechsten gasförmigen Produktstroms und (b) eines Phosgen enthaltenden sechsten flüssigen Produktstroms;
wobei
(VII)
(a) der in Schritt (I) anfallende erste flüssige Produktstrom mit einem gasförmigen Chlorwasserstoffstrom behandelt und dann direkt dem Schritt (II) oder dem Schritt (III) zugeführt wird,
(b) das im Reaktor zur Carbaminsäurechlorid-Spaltung aus Schritt (II) gebildete erste Reaktionsgemisch mit einem gasförmigen Chlorwasserstoffstrom behandelt wird,
(c) der in Schritt (II) anfallende zweite flüssige Produktstrom mit einem gasförmigen Chlorwasserstoffstrom behandelt und dann direkt dem Schritt (III) zugeführt wird,
(d) das in der Entphosgenierungsvorrichtung aus Schritt (III) gebildete zweite Reaktionsgemisch mit einem gasförmigen Chlorwasserstoffstrom behandelt wird
oder
(e) der in Schritt (III) anfallende dritte flüssige Produktstrom mit einem gasförmigen Chlorwasserstoffstrom behandelt und dann direkt dem Schritt (IV) zugeführt wird,
wobei das Behandeln mit dem gasförmigen Chlorwasserstoffstrom einstufig in einer Blasensäule oder in einer Bodenkolonne durchgeführt wird, wobei eine Kontaktzeit des gasförmigen Chlorwasserstoffstroms mit dem zu behandelnden flüssigen Produktstrom bzw. dem zu behandelnden Reaktionsgemisch im Bereich von 1 min bis weniger als 30 min eingestellt wird.

2. Verfahren gemäß Anspruch 1, bei welchem Schritt (VII) gemäß (VII)(a), (VII)(c) oder (VII)(e) durchgeführt wird.

3. Verfahren gemäß Anspruch 1, bei welchem Schritt (VII) gemäß (VII)(a) oder (VII)(c) durchgeführt wird.

4. Verfahren gemäß einem der vorstehenden Ansprüche, bei welchem der dritte flüssige Produktstrom Phosgen in einem auf seine Gesamtmasse bezogenen Massenanteil im Bereich von 0,001 ppm bis 1000 ppm enthält.

5. Verfahren gemäß einem der vorstehenden Ansprüche, bei welchem der in Schritt (VII) eingesetzte gasförmige Chlorwasserstoffstrom Phosgen in einem auf seine Gesamtmasse bezogenen Massenanteil im Bereich von 1 ppm bis 10000 ppm enthält.

6. Verfahren gemäß einem der vorstehenden Ansprüche, wobei das Verfahren den Schritt (VI) umfasst und wobei der sechste gasförmige Produktstrom teilweise oder vollständig in Schritt (VII) als gasförmiger Chlorwasserstoffstrom verwendet wird.

7. Verfahren gemäß einem der vorstehenden Ansprüche, bei welchem das molare Verhältnis von Chlorwasserstoff, der in dem in Schritt (VII) eingesetzten gasförmigen Chlorwasserstoffstrom enthalten ist, zu Isocyanatgruppen, die in dem in Schritt (VII) behandelten Reaktionsgemisch oder Produktstrom enthalten sind, n(HCl)/n(NCO), auf einen Wert im Bereich von 0,1 bis 2,0 eingestellt wird.

8. Verfahren gemäß einem der vorstehenden Ansprüche, bei welchem in Schritt (VII)
• der gasförmige Chlorwasserstoffstrom und
• der zu behandelnde Produktstrom bzw. das zu behandelnde Reaktionsgemisch im Gegenstrom geführt werden.

9. Verfahren gemäß einem der Ansprüche 1 bis 7, bei welchem in Schritt (VII)
• der gasförmige Chlorwasserstoffstrom und
• der zu behandelnde Produktstrom bzw. das zu behandelnde Reaktionsgemisch im Gleichstrom geführt werden.

10. Verfahren gemäß einem der vorstehenden Ansprüche, bei welchem die Behandlung mit dem gasförmigen Chlorwasserstoffstrom in Schritt (VII) bei einer Temperatur des zu behandelnden Produktstroms bzw. des zu behandelnden Produktgemisches im Bereich von 70 °C bis 135 °C und bei einer Temperatur des eingesetzten gasförmigen Chlorwasserstoffstroms im Bereich von 20 °C bis 135 °C durchgeführt wird.

11. Verfahren gemäß einem der vorstehenden Ansprüche bei welchem die Behandlung mit dem gasförmigen Chlorwasserstoffstrom in Schritt (VII) isotherm durchgeführt wird.

12. Verfahren gemäß einem der vorstehenden Ansprüche bei welchem in Schritt (VII) eine Kontaktzeit des gasförmigen Chlorwasserstoffstroms mit dem zu behandelnden Produktstrom bzw. dem zu behandelnden Reaktionsgemisch im Bereich von 1 min bis 25 min eingestellt wird.

13. Verfahren gemäß Anspruch 12, bei welchem in Schritt (VII) eine Kontaktzeit des gasförmigen Chlorwasserstoffstroms mit dem zu behandelnden Produktstrom bzw. dem zu behandelnden Reaktionsgemisch im Bereich von 1 min bis 20 min eingestellt wird.

14. Verfahren gemäß einem der vorstehenden Ansprüche, bei welchem die Behandlung mit dem gasförmigen Chlorwasserstoffstrom in Schritt (VII) bei einem Druck im Bereich von Umgebungsdruck bis 5,00 bar_{(abs.)} erfolgt.

15. Verfahren gemäß Anspruch 14, bei welchem die Behandlung mit dem gasförmigen Chlorwasserstoffstrom in Schritt (VII) bei einem Druck im Bereich von Umgebungsdruck bis 3,5 bar_{(abs.)} erfolgt.

## Claims

1. Process for preparing di- and polyisocyanates of the diphenylmethane series, comprising the steps of:
(I) reacting di- and polyamines of the diphenylmethane series with phosgene in the presence of a solvent in a phosgenation reactor to obtain (a) a first gaseous product stream containing hydrogen chloride and phosgene and (b) a first liquid product stream containing di- and polyisocyanates of the diphenylmethane series, phosgene and solvent;
(II) optionally reacting the first liquid product stream in a reactor for carbamoyl chloride cleavage to form in the reactor for carbamoyl chloride cleavage a first reaction mixture which is separated into (a) a second gaseous product stream containing hydrogen chloride and phosgene and (b) a second liquid product stream containing isocyanate, phosgene and solvent;
(III) separating phosgene from the first liquid product stream or, if step (II) is performed, from the second liquid product stream in a dephosgenation apparatus to form in the dephosgenation apparatus a second reaction mixture which is separated into (a) a third gaseous product stream containing hydrogen chloride and phosgene and (b) a third liquid product stream containing isocyanate and solvent;
(IV) separating solvent from the third liquid product stream to obtain (a) a fourth gaseous product stream containing solvent and (b) a fourth liquid product stream containing isocyanate, wherein the separation of the solvent is not followed by an inert gas treatment of the fourth liquid product stream obtained;
(V) optionally separating a portion of the diisocyanates of the diphenylmethane series from the fourth liquid product stream to obtain (a) a fifth gaseous product stream containing diisocyanates of the diphenylmethane series, which is condensed, and (b) a fifth liquid product stream enriched in polyisocyanates of the diphenylmethane series relative to the fourth liquid product stream;
(VI) optionally separating phosgene from the first and third gaseous product stream, if performing step (II) from the first, second and third gaseous product stream, to obtain (a) a sixth gaseous product stream containing hydrogen chloride and (b) a sixth liquid product stream containing phosgene;
wherein
(VII)
(a) the first liquid product stream obtained in step (I) is treated with a gaseous hydrogen chloride stream and then supplied directly to step (II) or step (III),
(b) the first reaction mixture formed in the reactor for carbamoyl chloride cleavage from step (II) is treated with a gaseous hydrogen chloride stream,
(c) the second liquid product stream obtained in step (II) is treated with a gaseous hydrogen chloride stream and then supplied directly to step (III),
(d) the second reaction mixture formed in the dephosgenation apparatus from step (III) is treated with a gaseous hydrogen chloride stream
or
(e) the third liquid product stream obtained in step (III) is treated with a gaseous hydrogen chloride stream and then supplied directly to step (IV),
wherein the treatment with the gaseous hydrogen chloride stream is performed in a single stage in a bubble column or in a tray column, wherein a contact time of the gaseous hydrogen chloride stream with the liquid product stream to be treated or the reaction mixture to be treated in the range from 1 min to less than 30 min is established.

2. Process according to Claim 1, wherein step (VII) is performed according to (VII)(a), (VII)(c) or (VII) (e) .

3. Process according to Claim 1, wherein step (VII) is performed according to (VII)(a) or (VII)(c).

4. Process according to any of the preceding claims, wherein the third liquid product stream contains phosgene in a mass fraction based on its total mass in the range from 0.001 ppm to 1000 ppm.

5. Process according to any of the preceding claims, wherein the gaseous hydrogen chloride stream employed in step (VII) contains phosgene in a mass fraction based on its total mass in the range from 1 ppm to 10 000 ppm.

6. Process according to any of the preceding claims, wherein the process comprises step (VI) and wherein the sixth gaseous product stream is partially or completely used as the gaseous hydrogen chloride stream in step (VII).

7. Process according to any of the preceding claims, wherein the molar ratio of hydrogen chloride present in the gaseous hydrogen chloride stream employed in step (VII) to isocyanate groups present in the reaction mixture or product stream treated in step (VII), n(HCl)/n(NCO), is set to a value in the range from 0.1 to 2.0.

8. Process according to any of the preceding claims,
wherein
• the gaseous hydrogen chloride stream and
• the product stream to be treated or the reaction mixture to be treated
are run in countercurrent in step (VII).

9. Process according to any of Claims 1 to 7, wherein
• the gaseous hydrogen chloride stream and
• the product stream to be treated or the reaction mixture to be treated
are run in cocurrent in step (VII).

10. Process according to any of the preceding claims, wherein the treatment with the gaseous hydrogen chloride stream in step (VII) is performed at a temperature of the product stream to be treated or of the product mixture to be treated in the range from 70°C to 135°C and at a temperature of the gaseous hydrogen chloride stream employed in the range from 20°C to 135°C.

11. Process according to any of the preceding claims, wherein the treatment with the gaseous hydrogen chloride stream in step (VII) is performed isothermally.

12. Process according to any of the preceding claims, wherein a contact time of the gaseous hydrogen chloride stream with the product stream to be treated or the reaction mixture to be treated in the range from 1 min to 25 min is established in step (VII).

13. Process according to Claim 12, wherein a contact time of the gaseous hydrogen chloride stream with the product stream to be treated or the reaction mixture to be treated in the range from 1 min to 20 min is established in step (VII).

14. Process according to any of the preceding claims, wherein the treatment with the gaseous hydrogen chloride stream in step (VII) is carried out at a pressure in the range from ambient pressure to 5.00 bar_{(abs.)}.

15. Process according to Claim 14, wherein the treatment with the gaseous hydrogen chloride stream in step (VII) is carried out at a pressure in the range from ambient pressure to 3.5 bar_{(abs.)}.

## Revendications

1. Procédé de préparation de di- et de polyisocyanates de la série du diphénylméthane, comprenant les étapes :
(I) réaction de di- et de polyamines de la série du diphénylméthane avec du phosgène en présence d'un solvant dans un réacteur de phosgénation avec obtention (a) d'un premier courant de produits gazeux, contenant du chlorure d'hydrogène et du phosgène, et (b) d'un premier courant de produits liquides, contenant des di- et des polyisocyanates de la série du diphénylméthane, du phosgène et un solvant ;
(II) en option, réaction d'un premier courant de produits liquides dans un réacteur pour dissociation du chlorure de carbamoyle, avec formation, dans le réacteur de dissociation du chlorure de carbamoyle, d'un mélange réactionnel qui est séparé (a) en un deuxième courant de produits gazeux contenant du chlorure d'hydrogène et du phosgène, et (b) en un deuxième courant de produits liquides contenant un isocyanate, du phosgène et un solvant ;
(III) séparation du phosgène du premier courant de produits liquides ou, lors de la mise en oeuvre de l'étape (II), du deuxième courant de produits liquides, dans un dispositif de déphosgénation, avec dans le dispositif de déphosgénation formation d'un deuxième mélange réactionnel qui est séparé (a) en un troisième courant de produits gazeux contenant du chlorure d'hydrogène et du phosgène et (b) en un troisième courant de produits liquides contenant un isocyanate et un solvant ;
(IV) séparation du solvant du troisième courant de produits liquides avec obtention (a) d'un quatrième courant de produits gazeux contenant un solvant et (b) d'un quatrième courant de produits liquides contenant un isocyanate, la séparation du solvant n'étant suivie d'aucun traitement par un gaz inerte du quatrième courant de produits liquides obtenu ;
(V) en option, séparation d'une partie des diisocyanates de la série du diphénylméthane du quatrième courant de produits liquides avec obtention (a) d'un cinquième courant de produits gazeux contenant des diisocyanates de la série du diphénylméthane, lequel est condensé, et (b) d'un cinquième courant de produits liquides, enrichi en polyisocyanates de la série du diphénylméthane par rapport au quatrième courant de produits liquides ;
(VI) en option, séparation du phosgène du premier et du troisième courants de produits gazeux, lors de la mise en oeuvre de l'étape (II) du premier, du deuxième et du troisième courants de produits gazeux, avec obtention (a) d'un sixième courant de produits gazeux contenant du chlorure d'hydrogène et (b) d'un sixième courant de produits liquides contenant du phosgène ;
dans lequel
(VII)
(a) le premier courant de produits liquides obtenu dans l'étape (I) est traité avec un courant de chlorure d'hydrogène gazeux, puis est directement envoyé à l'étape (II) ou à l'étape (III),
(b) le premier mélange réactionnel formé dans le réacteur de dissociation du chlorure de carbamoyle de l'étape (II) est traité avec un courant de chlorure d'hydrogène gazeux,
(c) le deuxième courant de produits liquides obtenu dans l'étape (II) est traité avec un courant de chlorure d'hydrogène gazeux puis est directement envoyé à l'étape (III),
(d) le deuxième mélange réactionnel formé dans le dispositif de déphosgénation de l'étape (III) est traité avec un courant de chlorure d'hydrogène gazeux
ou
(e) le troisième courant de produits liquides obtenu dans l'étape (III) est traité avec un courant de chlorure d'hydrogène gazeux, puis est directement envoyé à l'étape (IV),
le traitement avec le courant de chlorure d'hydrogène gazeux étant réalisé en une étape dans une tour de fractionnement à calottes ou dans une colonne à plateaux, avec ajustement d'un temps de contact dans la plage de 1 min à moins de 30 min du courant de chlorure d'hydrogène gazeux avec le courant de produits liquides à traiter ou avec le mélange réactionnel à traiter.

2. Procédé selon la revendication 1, dans lequel l'étape (VII) est mise en oeuvre selon (VII) (a), (VII) (c) ou (VII) (e).

3. Procédé selon la revendication 1, dans lequel l'étape (VII) est mise en oeuvre selon (VII) (a) ou (VII) (c).

4. Procédé selon l'une des revendications précédentes, dans lequel le troisième courant de produits liquides contient du phosgène selon une proportion en masse, rapportée à sa masse totale, dans la plage de 0,001 ppm à 1 000 ppm.

5. Procédé selon l'une des revendications précédentes, dans lequel le courant de chlorure d'hydrogène gazeux utilisé dans l'étape (VII) contient du phosgène selon une proportion en masse, rapportée à sa masse totale, dans la plage de 1 ppm à 10 000 ppm.

6. Procédé selon l'une des revendications précédentes, le procédé comprenant l'étape (VI), et le sixième courant de produits gazeux est en totalité ou en partie utilisé dans l'étape (VII) en tant que courant de chlorure d'hydrogène gazeux.

7. Procédé selon l'une des revendications précédentes, dans lequel le rapport en moles du chlorure d'hydrogène qui est contenu dans le courant de chlorure d'hydrogène gazeux utilisé dans l'étape (VII), aux groupes isocyanate qui sont contenus dans le mélange réactionnel ou dans le courant de produits traité dans l'étape (VII), n(HCl)/n(NCO), est ajusté à une valeur dans la plage de 0,1 à 2,0.

8. Procédé selon l'une des revendications précédentes, dans lequel dans l'étape (VII)
• le courant de chlorure d'hydrogène gazeux et
• le courant de produits à traiter ou le mélange réactionnel à traiter, sont guidés à contre-courant.

9. Procédé selon l'une des revendications 1 à 7, dans lequel, dans l'étape (VII)
• le courant de chlorure d'hydrogène gazeux et
• le courant de produits à traiter ou le mélange réactionnel à traiter, sont guidés en parallèle.

10. Procédé selon l'une des revendications précédentes, dans lequel le traitement avec le courant de chlorure d'hydrogène gazeux de l'étape (VII) est mis en oeuvre à une température du courant de produits à traiter ou du mélange de produits à traiter dans la plage de 70 °C à 135 °C et à une température du courant de chlorure d'hydrogène gazeux utilisé dans la plage de 20 °C à 135 °C.

11. Procédé selon l'une des revendications précédentes, dans lequel le traitement avec le courant de chlorure d'hydrogène gazeux de l'étape (VII) est mis en oeuvre dans des conditions isothermes.

12. Procédé selon l'une des revendications précédentes, dans lequel dans l'étape (VII) on ajuste un temps de contact du courant de chlorure d'hydrogène gazeux avec le courant de produits à traiter ou avec le mélange réactionnel à traiter dans la plage de 1 min à 25 min.

13. Procédé selon la revendication 12, dans lequel dans l'étape (VII) on ajuste dans la plage de 1 min à 20 min un temps de contact du courant de chlorure d'hydrogène gazeux avec le courant de produits à traiter ou avec le mélange réactionnel à traiter.

14. Procédé selon l'une des revendications précédentes, dans lequel le traitement avec le courant de chlorure d'hydrogène gazeux de l'étape (VII) est réalisé sous une pression dans la plage allant de la pression ambiante à 5,00 bar_{(abs.)}.

15. Procédé selon la revendication 14, dans lequel le traitement avec le courant de chlorure d'hydrogène gazeux de l'étape (VII) a lieu sous une pression dans la plage comprise entre la pression ambiante et 3,5 bar_{(abs.)}.
